# EUROPEAN PATENT SPECIFICATION

(11) **EP 0 738 342 B1**
(45) Date of publication and mention of the grant of the patent: **25.08.1999**
(21) Application number: 94909389.2
(22) Date of filing: 02.03.1994
(51) Int. Cl.: D21C 7/12, G01N 9/00, G01N 33/46

(54) **METHOD FOR CONTROLLING A PROCESS BY MEASUREMENT OF WOOD CHIPS**
VERFAHREN ZUR KONTROLLE EINES VERFAHRENS DURCH HOLZSCHNITZELMASS
PROCEDE DE REGULATION D'UN PROCEDE PAR MESURE DES COPEAUX DE BOIS

(30) Priority: 02.03.1993 SE 9300696
(43) Date of publication of application: 23.10.1996
(73) Proprietor: IGGESUND TOOLS AB, S-825 00 Iggesund (SE)
(72) Inventor: BÄCKLUND, Torbjörn, S-825 30 Iggesund (SE)
(74) Representative: Sedvall, Bengt Gustaf
(86) International application number: SE9400178
(87) International publication number: WO9420671

(56) References cited:
- SE-B- 460 383
- US-A- 4 239 590

## Description

The present invention relates to a control method for a defibration process by means of measuring wood chips.

For example, in a continous process for digesting cellulose for producing pulp the chips are supplied to the digester by means of chip wheel, such as a feeding-out valve, or by means of a screw. By adjusting the rotation speed of the chip wheel or the screw the parameters for the digesting process are determined and this adjustment is based on the chip bulk density being constant. Should the chip density vary, the digesting process will be operating with wrong parameters, thereby influencing the production yield, the digesting result, the pulp quality, the consumption of chemicals and similar and also influencing the treatments following the digesting process, e.g. bleaching. This may also result in a negative influence on the environment.

To be able to adjust the rotation speed of the chip wheel or the screw laboratory tests are carried out of the chips, during which a chip sample is taken and the chip bulk density is determined by means of any known method, such as volumetric determination, weighing and determination of moisture (see e.g. US-A-4239590). The chip bulk density being determined the received result is used to adjust the rotation speed of the chip wheel or the screw according to the description above and the digesting process or similar is proceeding under the condition that the chip bulk density remains unchanged. Such laboratory tests are usually performed once or several times a year. As mentioned previously, the chip bulk density is however seldom constant within one and the same kind of wood during such a long period and will vary substantially down to periods of parts of a day or an hour and the digesting process will therefore be performed partly under wrong assumptions.

The main purpose of the present invention is to propose a method to control a cellulose manufacturing process or similar, during which process considerations are made to fluctuations or variations in the chip bulk density in such a way as to continously adapt the process to the changes of the chip bulk density which may occur during the period of the digesting process.

Another purpose is to propose a method, with which samples can be continously taken out from the chips being supplied to the process and to measure from these samples all chip particles as to length, width and thickness and thereby determining the chip bulk density.

A further purpose of the invention is to propose a method of the kind mentioned previously, with which the obtained result of the chip bulk density can be presented as a result on a computer screen, representing a control signal for a continous calibration of the incoming chip bulk density in the process.

These and further purposes of the invention are achieved by means of a control method for a production process for cellulose, pulp or similar by means of measuring the bulk density of the chips supplied to the process, during which a representative volume of the delivered chips is sampled and is conveyed to a measuring method to measure the selected volume concerning the chip particle dimensions to calculate the chip bulk density by means of the measured values and to use the calculated chip bulk density to control the following chip treatment in the process.

According to the invention the calculated bulk density can be sent to a control program for the process as an input signal to adjust the process parameters to the eventually changed values of the chip bulk density. The calculated bulk density can be used to calculate the amount of wood being filled into a container when a certain filling level has been reached, such as in a batch digester, a chip lorry or similar.

The calculated bulk density can be used in combination with a weight and volume determination of the chips to detrmine the dry content of the chips, then being sent to a control program to adjust the process parameters to eventually changed values of the chip dry content/moisture.

Some of the measured chip dimensions or the chip composition and distribution can be used to control the process.

The samples are conveniently sampled with such a interval-time as to maintain a continous measuring method of all the chip particles incorporated in the chip sample. Before measuring the chip particles are separated from each other in the sampled chip volume, e.g. by means of vibration or similar and each chip particle in the sampled chip volume is measured as to length, width and thickness.

The calculated bulk density is used as a signal to control a chip wheel, a screw or a similar device, with which the chips are supplied to the process.

The invention will be described in the following by an embodiment, which however is meant only to illustrate the invention, other embodiments and applications being possible within the scope of the claims to follow.

As mentioned previously, several known methods do exist to determine the chip bulk density representing time consuming laboratory tests and thereby providing results which cannot be used to directly control or calibrate the process.

In one known method a by volume defined chip mass is weighed, whereafter some of the chips are dried to determine the moisture. Thereafter the bulk density can be determined or calculated.

In another method the chip diagonal/thickness ratio is determined by screening the chips in two kind of screens, one with which the largest dimension, i.e. the diagonal, is determined and one screen to determine the thickness. Thereafter the chip density can be determined.

It is obvious that these methods are not conveninet to continously determine the chip bulk density.

In contrast to these known methods samples are taken in the embodiment according to the invention of the chips being supplied to the process, for example each five minutes or at another convenient interval-time enabling the chips to be sent in a continous stream to a measuring method being described later. Thus, the interval-time between sampling is determined mainly by the time needed for the measuring process so that a continous measuring method of all the chip particles contained in the chip sample can be maintained. The thus regularly sampled chip samples, being e.g. sampled by separation from a conveyor belt transporting the chips to the cellulose process, have then to be separated, this being performed by e.g. separating the chip particles from each other by means of vibration and spreading them on a bed, such as another conveyer belt, on which they will be transported to the measuring method. During the measuring method each chip particle will be measured as to length, width and thickness, the chip bulk density then possibly to be calculated. The measuring and the calculation of the chip bulk density can be performed by means of some kind of chip analyzer being linked to a unit, in which the calculated chip bulk density value can be used for any further chip treatment in the cellulose process. The calculated value of the chip bulk density can thus be presented as a result on a computer screen for further computation of the chip qualities, such as e.g. the calculation of the chip moisture to be described later.

The calculated value of the chip bulk density can even be used as a control signal to continously calibrate the incoming chip bulk density in the process. The calculated value is then used for example to control the device, with which the chips are supplied to the process, such as a chip measuring wheel, a screw or a similar device, e.g. by regulating the rotating speed of the device.

The chip or wood moisture is important to know to optimize the process as to the product quality, consumption of chemicals, economy, etc. One tries to maintain a certain fluid/wood-ratio in the process. The chip moisture is nowadays measured by laboratory means (off line) with variating frequency. Such an analysis, however, is too slow for a direct process control, being performed by means of weighing-drying-weighing. There do even exist on-line meters being based on radioactive measuring, radio waves and microwaves or infrared optical systems, these methods, however, having all their specific problems and limits.

By determing the chip bulk density in accordance with the invention the chip moisture can even be calculated in a simple way by measuring the weight and the volume without adding any further complicated measuring equipment. Thus, the volume is easily measured by measuring the chip profile height in combination with a reading of the belt speed of the belt, with which the chips are transported and the weight is obtained by means of a belt scale for the same belt. The volume can also be determined by a direct measuring in the batch digester, the chip lorry, the feeding-out valve or similar.

By means of the values calculated from the chip dimensions of the chip bulk density a control parameter is created not being available previously, with which the process can be controlled for an optimal yield. The measuring procedure can be performed anywhere, where large chip volumes are handled, such as after the cutter or at a chip terminal, a digester or a refiner or somewhere else, where the best measuring place can be assumed to exist in the plant.

As an alternative to control the process by means of only the bulk density there is within the scope of the invention even the possibility to use the measured chip dimensions together with the chip composition or distribution for said control.

The control according to above can of course even be used to control the chip process up-streams the measuring place, such as chip deloading, cutting, chip mixing, screening, etc., the measuring being preferably arranged at a convenient place in the chip handling process.

By sampling a representative test chip volume regularly in this way, being as a whole completely measured as to the individual chip particle dimensions, a safe result will be obtained of the chip bulk density to be used directly or indirectly to control the process the chips are supplied to. A chip analysis comprises all forms of chips, whereby the measuring result becomes very accurate.

It is understood that the method according to the invention can be used to control other processes than the digester process described above, such as mechanical or chemical processes or any other process existing in this kind of industry and that the invention also is intended to comprise any process within the scope of the claims to follow.

## Claims

1. A method for measuring the bulk density of wood chips supplied to a process for manufacturing cellulose **characterized by** the steps of:
(a) sampling within a certain time interval a representative volume of the cellulose wood chips being supplied to the process and separating the wood chips from each other
(b) transporting the separated wood chips to a chips analyzer and measuring the dimensions of each of the chips contained in the sample volume;
(c) calculating the chip bulk density utilizing the information obtained in (b); and
(d) utilizing the calculated chip bulk density to control the treatment of the chips during the process of acting on the chips to produce cellulose pulp.

2. A method according to claim 1, **characterized in** that step (d) is practiced by sending information about the calculated chip bulk density to a control program which adapts process parameters to eventually change the chip bulk density values calculated from subsequent samples.

3. A method according to claim 1, **characterized in** that step (d) is practiced to calculate the amount of wood chips to be supplied to a chip container to obtain a certain fill level therein, for feeding chips to a batch digester or a chip lorry.

4. A method according to claim 1, **characterized of** the further step of determining the weight and volume of the wood chips; and utilizing the calculated bulk density from step (c), with the determined weight and volume information, to determine chip moisture/dry content.

5. A method according to claim 4, **characterized of** the further step of using the determined chip moisture/dry content as an input signal to a control program, and controlling the process for producing cellulose pulp with the control program to eventually change the chip's moisture/dry content for subsequent volumes for which it is calculated.

6. A method according to claim 1 **characterized in** that step (a) is practiced at such time intervals as to maintain a substantially continuous measuring of the chip dimensions of the chip samples.

7. A method according to claims 1-6, **characterized in** that the chip particles in the sampled chip volume are separated from each other before measuring, e.g. by means of vibration or similar.

8. A method according to anyone of claims 1-7, **characterized in** that each chip particle in the sampled chip volume is measured as to length, width and thickness.

9. A method according to anyone of claims 1-7, **characterized in** that the calculated bulk density or the measured chip dimensions or the chip composition and distribution is used as a signal to control a chip measuring wheel, a screw or a similar device, with which the chips are supplied to the process.

10. A method according to anyone of claims 1-9, **characterized in** that the process consists of a digesting process, a chemical or mechanical defibration process or similar.

11. A method according to anyone of claims 1-9, **characterized in** that the calculated bulk density or the measured chip dimensions or the chip composition and distribution is used to control the chip treatment in the process before the measuring place, after the measuring place, or both.

## Patentansprüche

1. Verfahren zum Messen der Festkörperdichte von Holzschnitzeln, die in einem Herstellprozeß für Zellulose zugegeben werden, gekennzeichnet durch folgende Schritte:
a) Probennahme in einem gewissen Zeitintervall eines repräsentativen Volumens von Zelluloseholzschnitzeln, die in dem Prozeß zugeführt werden, und Trennen der Holzschnitzel voneinander,
b) Transport der getrennten Holzschnitzel zu einem Schnitzelanalysator und Messen der Abmessung jedes im Probevolumen enthaltenen Schnitzels,
c) Berechnen der Schnitzelfestkörperdichte mittels der in b) erhaltenen Daten und
d) Verwendung der berechneten Schnitzelfestkörperdichte zur Regelung der Behandlung der Schnitzel beim auf die Schnitzel wirkenden Prozeß zur Herstellung von Zellulosebrei.

2. Verfahren nach Anspruch 1, dadurch gekennzeichnet, daß Schritt d) durchgeführt wird, indem Daten über die berechnete Schnitzelfestkörperdichte an ein Steuerprogramm gesendet werden, das Prozeßparameter an entsprechende Änderungen der Schnitzelfestkörperdichtewerte, die aus aufeinanderfolgenden Proben berechnet wurde, anpaßt.

3. Verfahren nach Anspruch 1, dadurch gekennzeichnet, daß Schritt d) durchgeführt wird, um die Menge an Holzschnitzeln zu berechnen, die einem Schnitzelbehälter zum Erreichen eines bestimmten Füllstandes zugeführt werden müssen, um sie einem Los-Zellstoffkocher oder einem Schnitzelförderwagen zuzuführen.

4. Verfahren nach Anspruch 1, gekennzeichnet durch einen Schritt der Bestimmung des Gewichtes und des Volumens der Holzschnitzel und die Verwendung der berechneten Festkörperdichte aus Schritt c) mit den erhaltenen Gewichts- und Volu-mendaten, um den Feuchtigkeitsgehalt bzw. Trocknungsgrad der Schnitzel zu bestimmen.

5. Verfahren nach Anspruch 4, gekennzeichnet durch den Schritt der Verwendung des festgestellten Feuchtigkeitsgehaltes bzw. Trocknungsgrades der Schnitzel als Eingangssignal für ein Steuerprogramm und Steuerung des Prozesses zur Herstellung von Zellulosebrei mit einem Steuerprogramm um den Feuchtigkeitsgehalt bzw. den Trocknungsgrad der Schnitzel für nachfolgende Volumen zu verändern, für die er berechnet wurde.

6. Verfahren nach Anspruch 1, dadurch gekennzeichnet, daß Schritt a) in solchen Zeitintervallen durchgeführt wird, so daß eine im wesentlichen gleichförmige Messung der Schnitzelabmessungen aus den Schnitzelproben aufrechterhalten wird.

7. Verfahren nach Ansprüchen 1 mit 6, dadurch gekennzeichnet, daß die Schnitzelteilchen im Probenschnitzelvolumen voneinander vor der Messung z.B. durch Vibration oder ähnliches getrennt werden.

8. Verfahren nach einem der Ansprüche 1 mit 7, dadurch gekennzeichnet, daß jedes Schnitzelteilchen im Probenschnitzelvolumen hinsichtlich Länge, Breite und Dicke vermessen wird.

9. Verfahren nach einem der Ansprüche 1 mit 7, dadurch gekennzeichnet, daß die berechnete Festkörperdichte oder die gemessenen Schnitzelabmessungen oder die Schnitzelzusammensetzung und -verteilung als Signal zum Ansteuern eines Schnitzel-meßrades, einer Schraube oder einer ähnlichen Vorrichtung verwendet werden, mit der die Schnitzel dem Prozeß zugeführt werden.

10. Verfahren nach einem der Ansprüche 1 mit 9, dadurch gekennzeichnet, daß der Prozeß aus einem Kochprozeß, einem chemischen oder mechanischen Holzauf-schlußprozeß oder ähnlichem besteht.

11. Verfahren nach einem der Ansprüche 1 mit 9, dadurch gekennzeichnet, daß die berechnete Festkörperdichte oder die gemessenen Schnitzelabmessungen oder die Schnitzelzusammensetzung und -verteilung zur Steuerung der Schnitzelbehandlung im Prozeß vor der Meßstelle und/oder nach der Meßstelle verwendet wird.

## Revendications

1. Procédé de mesure de la masse volumique apparente de copeaux de bois transmis à une opération de fabrication de cellulose, caractérisé par les étapes suivantes :
(a) l'échantillonnage, au cours d'un certain intervalle de temps, d'un volume représentatif des copeaux de bois cellulosique transmis pour être traités, et la séparation des copeaux de bois les uns des autres,
(b) le transport des copeaux de bois séparés vers un analyseur de copeaux et la mesure des dimensions de chacun des copeaux contenus dans le volume d'échantillon,
(c) le calcul de la masse volumique apparente des copeaux à l'aide des informations obtenues en (b), et
(d) l'utilisation de la masse volumique apparente calculée des copeaux pour la commande du traitement des copeaux pendant le procédé qui agit sur les copeaux pour la production d'une pâte cellulosique.

2. Procédé selon la revendication 1, caractérisé en ce que l'étape (d) est mise en oeuvre par transmission d'informations relatives à la masse volumique apparente calculée des copeaux à un programme de commande qui adapte les paramètres de traitement pour modifier finalement les valeurs de masse volumique apparente de copeaux calculées à partir d'échantillons ultérieurs.

3. Procédé selon la revendication 1, caractérisé en ce que l'étape (d) est mise en oeuvre pour le calcul de la quantité de copeaux de bois destinés à être transmis à un récipient de copeaux pour l'obtention d'un certain niveau de remplissage dans le récipient afin que les copeaux soient transmis à un digesteur discontinu ou à un chariot à copeaux.

4. Procédé selon la revendication 1, caractérisé par une étape supplémentaire de détermination de la masse et du volume des copeaux de bois, et d'utilisation de la masse volumique apparente calculée dans l'étape (c) avec les informations déterminées de masse et de volume pour la détermination de la teneur en humidité et de la teneur en matières sèches des copeaux.

5. Procédé selon la revendication 4, caractérisé par une étape supplémentaire d'utilisation des teneurs déterminées en humidité et en matières sèches des copeaux comme signal d'entrée d'un programme de commande, et de commande du procédé de production de la pâte cellulosique avec le programme de commande qui modifie finalement les teneurs en humidité et en matières sèches des copeaux pour des volumes ultérieurs pour lesquels les teneurs sont calculées.

6. Procédé selon la revendication 1, caractérisé en ce que l'étape (a) est mise en oeuvre à certains intervalles de temps tels qu'une mesure pratiquement continue des dimensions des copeaux des échantillons est conservée.

7. Procédé selon l'une quelconque des revendications 1 à 6, caractérisé en ce que les particules des copeaux du volume échantillonné de copeaux sont séparées les unes des autres avant la mesure, par exemple par vibration ou un processus analogue.

8. Procédé selon l'une quelconque des revendications 1 à 7, caractérisé en ce que chaque particule des copeaux du volume échantillonné de copeaux est mesurée dans sa longueur, sa largeur et son épaisseur.

9. Procédé selon l'une quelconque des revendications 1 à 7, caractérisé en ce que la masse volumique apparente calculée ou les dimensions mesurées des copeaux ou la composition et la distribution des copeaux sont utilisées comme signal de commande d'une roue de mesure de copeaux, d'une vis ou d'un dispositif analogue avec lequel les copeaux sont transmis au processus de traitement.

10. Procédé selon l'une quelconque des revendications 1 à 9, caractérisé en ce que le processus de traitement est un processus de digestion, un processus de défibrage chimique ou mécanique ou un processus analogue.

11. Procédé selon l'une quelconque des revendications 1 à 9, caractérisé en ce que la masse volumique apparente calculée ou les dimensions mesurées des copeaux ou la composition et la distribution des copeaux sont utilisées pour le réglage du traitement des copeaux dans le procédé exécuté avant l'emplacement de mesure, après l'emplacement de mesure ou à ces deux emplacements.
